(19) Europäisches Patentamt
European Patent Office
Office européen des brevets

(11) EP 2 221 039 B1

(12) EUROPEAN PATENT SPECIFICATION

(45) Date of publication and mention of the grant of the patent:
**22.11.2017 Bulletin 2017/47**

(21) Application number: **09153068.3**

(22) Date of filing: **18.02.2009**

(51) Int Cl.:
*A61Q 15/00* (2006.01) *A61K 8/26* (2006.01)
*A61K 8/28* (2006.01) *A61K 8/11* (2006.01)
*A61K 8/84* (2006.01)

# (54) Antiperspirant compositions

Schweißhemmende Zusammensetzungen

Compositions anti-transpirantes

(84) Designated Contracting States:
**AT BE BG CH CY CZ DE DK EE ES FI FR GB GR HR HU IE IS IT LI LT LU LV MC MK MT NL NO PL PT RO SE SI SK TR**

(43) Date of publication of application:
**25.08.2010 Bulletin 2010/34**

(73) Proprietors:
- **Unilever PLC, A Company Registered in England and Wales under Company no. 41424**
  **London EC4Y 0DY**
  **Greater London (GB)**
  Designated Contracting States:
  **CY GB IE MT**
- **UNILEVER N.V.**
  **3013 AL Rotterdam (NL)**
  Designated Contracting States:
  **AT BE BG CH CZ DE DK EE ES FI FR GR HR HU IS IT LI LT LU LV MC MK NL NO PL PT RO SE SI SK TR**

(72) Inventors:
- **Chan, Catrin, Sian**
  **Leeds, LS14 2AR (GB)**
- **Cropper, Martin, Peter**
  **Wirral**
  **Merseyside CH63 3JW (GB)**
- **Franklin, Kevin, Ronald**
  **Wirral**
  **Merseyside CH63 3JW (GB)**
- **Johnson, Simon, Anthony**
  **Wirral**
  **Merseyside CH63 3JW (GB)**
- **McKeown, Robert**
  **Wirral**
  **Merseyside CH63 3JW (GB)**

(74) Representative: **Whaley, Christopher et al**
**Unilever Patent Group**
**Colworth House**
**Sharnbrook**
**Bedford MK44 1LQ (GB)**

(56) References cited:
**EP-A- 0 397 246** **EP-A- 1 407 754**
**EP-A- 1 797 947** **GB-A- 2 274 989**
**US-A- 3 516 941** **US-A- 5 135 747**

- **H. HERMA, E. BONATZ, K. GOLZ: "Einsatz von Aminoharz-Mikrokapseldispersionen in kosmetischen Präparaten" PARFÜMERIE UND KOSMETIK, vol. 72, no. 7, 1991, pages 434-438, XP009119302**
- **"Encapsulation / Microencapsulation Services Specifications", , 13 November 2012 (2012-11-13), Retrieved from the Internet: URL:http://www.aveka.com/particle-micro-bead-making.html**
- **E. N. Brown et al.: "In situ poly(urea-formaldehyde) microencapsulation of dicyclopentadiene", , 13 November 2012 (2012-11-13), Retrieved from the Internet: URL:https://www.ideals.illinois.edu/bitstream/handle/2142/281/1014.pdf?**
- **S. A. ODOM ET AL.: "Visual Indication of Mechanical Damage Using Core-Shell Microcapsules", APPL. MATER. INTERFACES, 24 November 2011 (2011-11-24), pages 4547-4551,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- K. DIETRICH ET AL.: "Amino resin microcapsules", ACTA POLYMERICA, vol. 40, no. 5, 1989, pages 325-331, XP055059144,

## Description

The present invention relates to antiperspirant compositions and more particularly to anhydrous contact compositions.

During the course of normal day to day activities, such as from physical activity or from being subjected to stress, mankind perspires, resulting, in the absence of remedial treatment, in damp patches on the skin or in clothing that comes into contact with such skin and in the generation of malodour arising from skin microflora acting upon secretions from inter alia eccrine glands. The distribution of eccrine glands is uneven in the skin, being concentrated particularly in the underarm. Consequently, the underarm is where damp patches and/or malodour generation is most prone to occur.

In many societies, body malodour is considered to be undesirable or even antisocial. Similarly, persons exhibiting damp patches can suffer embarrassment or even criticism. As a consequence, an industry has grown up to prevent or at least hinder sweating and to reduce the generation of body malodour by the creation of cosmetic antiperspirant compositions that are applied topically to skin, particularly in those localised parts of the body where the density of eccrine glands is greatest, such as in the underarm. Whilst bathing can remove sweat, it is not preventative and in any event, there is growing pressure on the world's water so that regular use of antiperspirants as part of a cleanliness regime helps to conserve water supplies. Moreover, the cost of heating water by gas boilers or by electricity generated from gas has increased substantially since 2000, so that regular bathing or showering is an increasingly expensive activity. Commonly, body malodour is counteracted by both washing and regular application of an antiperspirant composition.

The antiperspirant industry has recognised that it is convenient for users to apply an antiperspirant composition after washing, commonly in the bathroom or shower-room such as once or twice a day, for example in the morning or evening, or during preparation for a social gathering. It is less convenient to apply antiperspirant at work or during a social gathering, so that it would be desirable for the effectiveness of an antiperspirant composition to last for several hours, such as up to 24 hours after application.

It would also be reassuring for the user to be reminded periodically of the continuing effectiveness of the antiperspirant composition, removing the anxiety that itself could induce sweating. One of the means adopted by the industry to reduce the impact of malodour generation on the body is the incorporation of a fragrance or a plurality of fragrances into the antiperspirant composition. The fragrance can mask the body odour and some fragrances, so-called deo-fragrances, can also function as a bactericide or bacteristat preventing or retarding the formation of malodorous compounds on the skin.

It has long since been understood that a fragrance functions by being evaporated from the skin onto which it has been applied topically, so that after a while, its concentration falls below that at which it is olfactorily detected. The rate at which that will occur depends on the volatility of the perfume component and its inherent volatility, often approximated by its boiling point. The art of the perfumer is to select a blend of perfume components that satisfies the aesthetic criteria for that perfume. However, it has also been recognised that the perfumer can slow down the rate of evaporation of a perfume by encapsulating it, thereby prolonging the effective life of the perfume after it has been applied topically. Likewise, it was recognised that it was necessary for the perfume to be released from the capsules containing it. In other words, the encapsulation has to be reversible.

A number of encapsulating materials have been proposed for encapsulating perfumes, including modified starches and various gums, including acacia gum.

Such materials are water soluble at body temperature, so that the disruption of the capsule wall can be triggered by moisture, including, in particular, sweat. Whilst this has the advantage of being localised and triggered by a sweat event, it has the disadvantage that sweat, on production, is typically substantially odourless, whereas malodour is generated over an extended period of time by the action of localised microflora, such as Coryne bacteria transforming solutes within the excretion. Thus, the masking perfume can be released before the malodour is generated and there is a significant risk that it will have been dissipated before the malodour is fully generated. Accordingly, it would be desirable to employ an alternative encapsulating material, one that does not rely upon sweating to cause its wall to rupture.

Anhydrous contact compositions comprise some of the commonest types of antiperspirant composition, and especially sticks (firm solids) or creams (soft solids) in North America. Anhydrous antiperspirants are characterised by the absence of an aqueous liquid phase. Contact compositions are characterised by being applicable directly from a dispensing container into direct contact with skin.

Many antiperspirant salts, such as aluminium and/or zirconium halohydrate salts comprise a small fraction of bound water, possibly in the region of from 1 to 3% by weight of the antiperspirant salt, but such bound water is present within the solid salt particles and not as "free" water. Accordingly, compositions containing them are still deemed to be anhydrous. It would be particularly desirable to be able to devise anhydrous antiperspirant compositions containing an encapsulated perfume in which the capsule wall was disrupted by a means other than sweat.

It has been proposed to incorporate encapsulated perfume into aqueous fabric or hair conditioning compositions

employing an encapsulating material that is water-insoluble, and shear sensitive. The capsules are incorporated as an aqueous slurry and the resultant composition applied to a substrate, via an aqueous composition. Subsequently, the fragrance is released by the substrate being rubbed, or abraded, typically quite vigorously, thereby rupturing the shell of the encapsulate. The presence of water has often been considered to be of crucial importance, because the rupture-resistance of the capsule wall (the shell) is sensitive to moisture. In an aqueous medium, the shear-sensitive encapsulates are much more resistant to being ruptured. When the capsules are dry, their resistance is significantly diminished.

GB-A-2,274,989 (Firmenich & Cie, 1984) discloses non-aqueous perfuming compositions with antiperspirant action containing a perfuming base formed of two perfuming elements, one of which is in liquid form and the other of which is in water-soluble microencapsulated form.

It has been found during the course of research leading to the instant invention that powdery shear sensitive encapsulates containing a fragrance can be suspended effectively in a carrier liquid for the particulate antiperspirant active material comprising a hydrophobic liquid, commonly called an oil or a mixture of oils, in anhydrous antiperspirant compositions, provided that the encapsulates satisfy certain physical criteria. In such circumstances, a significant fraction of the encapsulates remain intact and capable of releasing the encapsulated contents after application of the antiperspirant composition onto skin, such as in the armpit. The perfume can then be released by lateral movement of clothing or other skin across and in contact with the surface of skin to which the composition was applied, or by impact of for example the arm on the chest during normal day-today movement of the arm.

If the encapsulates have physical characteristics outside defined ranges, pre-application retention or post-application release of fragrance is impaired. For example, if the capsules are too weak, they do not survive the manufacture process or the application process, whereas if they are too strong, greater pressure is needed to rupture them than is provided by one area of skin or clothing rubbing past the area of skin to which the composition has been applied or by normal impact of arm on chest.

Brief summary of the present invention

According to a first aspect of the present invention, there is provided an anhydrous contact antiperspirant composition according to claim 1. By the employment of such a dry particulate shear sensitive encapsulated perfume satisfying selected characteristics, it is possible to deposit on skin a residual fraction of shear-sensitive encapsulated perfume particles that can be ruptured by the passage of a garment across the surface of the skin or by the movement of one area of skin relative to another, such as in the underarm, at a time when sweating is or is not occurring or irrespective of whether sweating has occurred. Advantage is accordingly taken of the sensitivity of such a dry particle on the skin surface to be ruptured by relative movement of garment or skin to skin, but the presence of the thickened or gelled liquid carrier enables the significant fraction of the capsules to be so deposited from conventional contact applicators. This enables improved masking of malodour and enhanced perception of fragrance over a prolonged period.

Although it is possible for some encapsulates having characteristics outside the ranges identified herein to offer some residual fragrance release activity, for example encapsulates having a shell formed from starches or related water-soluble or dispersible materials, when triggered by encountering sweat, the selection of encapsulates satisfying the specified ranges according to the present invention combines manufacturing capability under the conditions for making anhydrous stick antiperspirant compositions with greater availability of releasable fragrance in the underarm. Also disclosed herein is the use of a composition according to claim 1 that simultaneously a) prevents or reduces localised sweating by topical application of a composition according to claim 1 and b) prolongs perception of perfume or masks body-generated malodour, even when sweating is not occurring or irrespective of whether sweating has occurred.

Detailed Description of the invention, including preferred embodiments.

The present invention relates to the incorporation into anhydrous antiperspirant contact compositions of shear sensitive encapsulated perfume capsules, the term capsules herein including microcapsules. Shear sensitive herein contemplates that the capsule is capable of releasing its perfume contents by rubbing of the upper arm forwards or backwards across in contact with the chest wall whilst remaining in contact with it or by the rubbing of clothing worn on the upper arm or chest likewise rubbing across skin in the upper arm or armpit to which the antiperspirant composition has been applied.

The encapsulating material for the shear-sensitive capsules herein is selected from aminoplast polymers, by which is meant resins obtained by polymerisation of an amine with an aliphatic aldehyde. The amine advantageously has a molecular weight of up to 150 and often at least 50. The aldehyde advantageously has a molecular weight of up about 60, such as about 30 or 45.

The proportion of shell material to core perfume oil is at the discretion of the producer, and is attainable by appropriately varying the proportions of the ingredients in the emulsion. It is desirable for the shell material to constitute from 30 to 80% of the capsules, and particularly from 35 to 60% by weight of the capsules. By varying the proportions

of shell and core, the physical strength of the shell can be varied (for capsules of the same weight average particle size). Accordingly, capsules having the desired combination of characteristics can be selected. The balance of the weight of the capsules is provided by the contents of the core, which comprises the fragrance oil, optionally together with a diluent oil.

The average core volume of the capsules is desirably at least 30% and advantageously at least 35%. In many embodiments, the core volume is up to 60% and especially up to 50%.

Advantageously, the fragrance oil is present in the core in a weight proportion of at least 20% of the capsules and especially at least 30% of the capsules. In many desirable embodiments, the weight proportion of fragrance oil in the capsules is up to 55% and particularly up to 50%. Commonly any diluent oil provides not more than 25% of the combined weight of fragrance plus diluent oil. The diluent oil can comprise any oil that is employed as a carrier oil in fragrance compositions.

It is preferred for the volume average particle diameter (size) of the capsules to be at least 33 μm and particularly at least 40 μm and in many desirable embodiments is up to 60 μm in diameter. Herein, unless otherwise indicated, the weight average particle diameter of the encapsulates (D[4,3]) is that obtainable using a Malvern Mastersizer, the encapsulates being dispersed in cyclopentasiloxane (DC245) using a dispersion module mixer speed of 2100 rpm. Calculations are made using the General Purpose model, assuming a spherical particle shape and at Normal calculation sensitivity. The shell thickness can be measured by solidifying a dispersion of the capsules in a translucent oil, cutting a thin slice of the solid mass and using a scanning electron microscope to obtain an image of cut-through individual capsules, thereby revealing the inner and outer outline of its annular shell and hence its thickness.

The shell thickness of the microcapsules tends to increase as the particle size increases. The shell thickness, accordingly, often ranges within the thickness of from 1 to 9μm. For capsules of diameter up to 40 μm, the shell thickness is often below 6 μm, such as from 1 to 5 μm whereas for particle of at least 40 μm the shell thickness is often from 4 or 5 to 10 μm. The average shell thickness is often in the range of from 4 to 8 μm, and particularly where the average capsule diameter is from 45 to 60 μm. In many desirable embodiments, the average shell thickness is at least 5 μm, such as in the region of 5.5 to 7 μm.

The hardness of the capsules, as measured in a Hysitron Triboindenter, is an important characteristic that enables them to be incorporated effectively in anhydrous formulations, retaining the capability of being sheared by frictional contact between skin and skin or clothing. The hardness is desirably in the range of from 0.5 to 50 MPa and especially from 2.5 up to 15 MPa, and in many embodiments is up to 10 MPa. In certain preferred embodiments, the hardness is in the range of from 2.5 to 4 MPa.

A further parameter of interest in relation to the capsules in the instant invention, and particularly their capability to be sheared by friction in the compositions and process of the instant invention, is their "Apparent Reduced Elastic Modulus (Er). Desirably, Er falls within the range of from 18 to 35 MPa, and in many convenient embodiments, in the range of from 20 to 25 MPa.

The hardness (Hysitron Hardness) and Apparent Reduced Elastic Modulus measurements shown herein are those measured by the following method:-A drop of a dispersion of the capsules in demineralised water is placed onto a piece of silicon wafer and allowed to dry leaving behind discrete micro encapsulates for mechanical analysis. The dried wafer is fitted into the Hysitron Triboindenter and spatially mapped using the optical system of the instrument to identify a perimeter around the sample.

The head of the Triboindenter is fitted with a Berkovich tip, a three sided pyramid, which compresses individual capsules. A single capsule is positioned directly under the Indenter tip. The instrument is programmed to perform an indent by compressing the sample with an initial contact force of 75 μN, for 10 seconds, followed by a position hold stage for 1 second and a decompression stage for 10 seconds. The instrument achieves a very small load (typically around 15-30 μN). The Hysitron Hardness (H in MPa) and reduced Elastic Modulus (Er in MPa) are calculated from the relaxation stage of the force deflection data using the following equations.

$$H = \frac{W}{A}$$

W = Compressive force
A = Contact Area ($A \approx 24.56 h_c^2$)

$$Er = \frac{\sqrt{\pi}}{2\gamma}\frac{S}{\sqrt{A}}$$

S = Contact Stiffness ($dW/dh_t$)

$h_t$ = Total Penetration Depth
$\gamma$ = 1.034

$$h_c = h_t - \kappa \frac{W}{S}$$

K = 3/4
$h_c$ = Contact Depth

Results are averages of a minimum of 20 measurements made on capsules with a particle size of D[4,3] +/- 20%

The aminoplast-shelled microcapsules employable in the instant invention can be made in accordance with the general process described in US3516941, in which a disclosure in regard to urea is representative of the amine reactant generally, including melamine in particular. In such a process, in a first step, a low molecular weight amine-aldehyde precondensate is formed, advantageously urea or melamine with formaldehyde, suitably in an equivalent mole ratio of amine:aldehyde of 1:1.2 to 2.6 and preferably about 1:2. Preferably the reaction is alkaline catalysed and conducted in an aqueous medium at a pH in the range of from 7.5 to 11.0, at an elevated temperature below 100°C and particularly from 50 to 90°C. The reaction is allowed to continue until the precondensate has formed to a desired extent, commonly selected in the range of from 15 minutes up to 3 hours, preferably correlating reaction time inversely with reaction temperature. The resultant precondensate is a water-soluble material that is dissolved in water at a concentration of from 3 to 30% by weight, preferably from 10 to 25% by weight.

The fragrance oil, together with any diluent oil if present, is introduced into the aqueous solution of the pre-condensate and subjected to intense mixing to form droplets that are distributed throughout the aqueous phase, the mixing intensity being controlled in a conventional manner so as to control the average diameter of the droplets. This stage of the process is conveniently conducted at a temperature of from 10 to 50°C, such as at ambient. It is desirable for the fragrance oil droplets to have an average diameter of from 25 to 55 μm, particularly at least 30 μm and in some especially desirable instances at least 35 μm. The dispersed droplets in some particularly suitable embodiments have a diameter of up to 45 μm.

The dispersion of the fragrance oil droplets in the precondensate solution is acidified by addition of acid to attain a solution pH in the region of from pH1 to pH5, in order to promote acid catalysis of the pre-condensate, with continued intense mixing of the solution. Particularly, the solution is acidified to below pH3.5 or pH3, and in many instances, the solution pH is not lower than pH1.5. This stage of the process is often conducted at a temperature in the range of from about 20°C to 90°C and for a period of at least 1 hour. It is convenient and desirable to conduct this stage initially at a lower temperature of below 40°C, eg ambient, for a period of for example 20 to 60 minutes and thereafter to increase the temperature of the reaction mixture to within the range of from 60 to 90°C for the remainder of the reaction period. The total reaction period is often not greater than 3 hours and in many instances is from 1.5 to 2.5 hours, and particularly including reaction at a temperature of at least 60°C for from 45 to 90 minutes. In the event that the temperature were to be maintained below 60°C throughout, the total reaction time is often up to 6 hours.

As a variation to acidification alone, the acid catalysis can be conducted in the additional presence of a mineral acid/alkali metal salt, such as sodium chloride, at a concentration of 2-20% by weight in the precondensate solution, and preferably at a pH in the range of from pH4 to pH5.

At the end of the acid catalysis, the capsules comprise a hard aminoplast shell surrounding a fragrance oil-containing core, forming an aqueous slurry. The aqueous phase is separated from the capsules, for example by filtration or centrifugation, and the capsules dried, for example until they have a residual moisture content of below 5% by weight and especially to below 3% by weight. Drying can be carried out by conventional means for sub 100 μm particles, such as by tray drying, commonly at a temperature of below 150°C and often below 125°C, in order to avoid volatilising the fragrance oil or a significant fraction of it. Alternatively, the drying can be conducted under vacuum.

If desired, the manufacture process can be modified in accordance with the description in EP1797947, namely the incorporation of a formaldehyde scavenger to, for example, the slurry containing the aminoplast capsules. The scavenger can be a beta dicarbonyl compound such as 1,3-cyclohexanedione, or manganese dioxide.

By control of the manufacturing process conditions, the resultant dry capsules having the characteristics specified in the ranges or preferred ranges for particles size and mean diameter described herein can be obtained.

The capsules, by virtue of their manufacture route often contain a small residual water content. It is desirable, for example, as measured by the conventional Karl Fischer method, to select capsules having a residual water content of below 5% by weight and particularly below 4% by weight, such as from 0.5 to 3.5% and particularly from 0.6 to 3% w/w (based on the fragrance-containing capsule). Based on the weight of the shell, said water content of the capsules employed herein often falls in the range of from 1% to 20% w/w. By limiting the proportion of water in the capsule, and particularly in the shell, it is possible to avoid at least partly, and preferably substantially, the formation of grit within the anhydrous formulation, and thereby avoid the negative sensation of grit on underarm skin. Grit occurs typically when particles aggregate to form agglomerates that are not readily fractured into their constituent particles. Accordingly, in regard to aerosol or spray compositions, the avoidance of grit formation has a second benefit of reducing the likelihood of blockage of the spray nozzle.

The shear sensitive encapsulate or mixture of encapsulates is (are) employed in the antiperspirant compositions in an amount from 0.5 to 2.5% by weight of the composition. Accordingly, the base compositions before introduction of propellant contain a proportionately higher proportion of the encapsulate.

The perfume oil employable herein can be selected as is conventional to attain the desired aesthetic result, and comprises usually a blend of at least 5 components, and often at least 20 components. The components can be synthetic or natural extractions, and, in the case of natural oils or oils produced to mimic natural oils, are often mixtures of individual perfume compounds. The perfume oil can comprise, inter alia, any compound or mixture of any two or more such compounds coded as an odour (2) in the Compilation of Odor and Taste Threshold Values Data edited by F A Fazzalari and published by the American Society for Testing and Materials in 1978.

Often, though not exclusively, the perfume compounds acting as perfume components or ingredients in blends have a ClogP (octanol/water partition coefficient) of at least 0.5 and many a ClogP of at least 1. Many of the perfume components that are employable herein can comprise organic compounds having an odour that is discernible by humans that are selected within the chemical classes of aldehydes, ketones, alcohols, esters, terpenes, nitriles and pyrazines. Mixtures of compounds within classes or from more than one class can be blended together to achieve the desired fragrance effect, employing the skill and expertise of the perfumer. As is well known, within the same class, those compounds having a lower molecular weight, often up to about 200, tend to have a lower boiling point and be classified as "top notes", whereas those having a higher molecular weight tend to have a higher boiling point and be classified as middle or base notes. The distinction, though, is to some extent an arbitrary simplification, because the fragrance oils form a continuum and their characteristics are not significantly different close to on either side of an arbitrary boundary such as a boiling point of 250°C or 275°C. Herein, the perfume can comprise any blend of oils boiling at below 250°C (such as in the range 1 to 99% or 4 to 96%, 10 to 90% or 25 to 60%) with the balance provided by compounds having a boiling point above 250°C. The perfumer recognises that the lower boiling point compounds tend to evaporate more quickly after exposure, whereas higher boiling point compounds tend to evaporate more slowly, so that the desired aesthetic effect can be achieved by selecting the proportions of the faster and slower compounds - the faster providing an instant "hit" whilst the slower providing a longer lasting impact. It will also be recognised that a term such as high impact has also been used to describe low boiling point perfume compounds. The properties of the compound stay the same irrespective of whether they are called high impact or top note ingredients.

A further characteristic of a perfume compound is its odour detection threshold (ODT). Some perfume oils are much more easily detected by the human nose than others, but it is a very subjective measurement and varies considerably depending on the way that testing is performed, the prevailing conditions and the make-up of the panel, e.g. age, gender and ethnicity. As a qualitative means of differentiating between the aesthetic attributes of compounds, and enabling the perfumer to choose ingredients that are detected relatively easily, the ODT represents a useful guide, but quantitatively is more dubious.

Examples of perfume compounds or oils that can be employed within the capsules described herein, ether singly, or more usually as a blend of at least 5 or at least 10 or at least 20 or at least 50 compounds listed hereinafter include:-1,1,6,7- tetramethyl naphthalene, 1-(2,6,6-trimethyl-1,3-cyclohexandienyl)-2-buten-1-one, 1-(5,5-dimethyl-1-cyclohexen-1-yl)-1h-indene-a-propanal, 1,2,3,4,5,6,7,8-octahydro-1,1,6,7-tetramethyl naphthalene, 1,3-oxathiane, 11-dodecatetraenal, 1-methyl-4-isopropenyl-1-cyclohexene, 2,3-dihydro-1,1-dimethyl-(9ci), 2,4-dimethyl 2,6,-nonenol, 2,4-dimethylcyclohexene-3-carbaldehyde, 2-n-heptyl cyclopentanone, 2,6-nonadienal, 2,6-dimethyl-2,6-octadien-8-ol, 2,6-dimethyl-5-heptenal, 2,6-nonadien-1-ol, 2-isobutylthiazole, 2-methoxy-3- (2-methylpropyl)- pyrazine, 2-methoxy-4-(2-propenyl)phenol, 2-methoxy-4-vinylphenol, 2-methyl-4-propyl-cis-paradiff, 2-propenyl ester, 3- methyl-4-(2,6,6-trimethyl-2- cyclohexen-1-yl) cyclogalbanate, 3-(3-isopropylphenyl)butanal, 3,3-dimethyl-5-(2,2,3-trimethyl-3-cycloenten-1-yl)-4-penten-2-ol, 3,6 nonadienol, 3,7-dimethyl-1,6-octadien-3-ol, 3,7-dimethyl-2, 3,7-dimethyl-2,6-octadienenitrile, 3,7-dimethyl-6-octen-1-ol, 3-buten-2-one, 3-cyclohexadienyl)-3-buten-4-one, 3-cyclohexene-1-carboxaldehyde, 3-methyl-2-buten-1-yl acetate, 3-methyl-4-(2,6,6-trimethyl-2-cyclohexen-1-yl), 3-p-cumenyl-propionaldehyde 4-(1-methylethyl)benzenepropanal, 4 dodecenal, 4-(2,6,6-trimethyl-2-cyclohexen-1-yl)(e), 4-methoxybenzaldehyde, 4-methyl-3-decen-5-ol, 4-methyl-4-mercaptopentan-2-one, 4-penten-1-one, 6-(z,3-pentenyl)-tetrahydro-(2h)-pyranone-2,and 3-methyl-(cis-2-penten-1-yl)-2-cyclopenten-1-one., 6-octadienal, 6-trimethyl-1, 7-acetyl 1 2 3 4 5 6 7 8- octahydro-1, 8-cyclohexadecen-1-one, acetic acid (cyclohexyloxy,2-propenyl ester, acetyl cedrene, acetyl tributly citrateadoxal, aldehyde supra, allyl amyl glycolate, allyl caproate, allyl cyclohexane propionate, allyl heptanoate, allyl heptoate, alpha damascone , alpha methyl ionone iso, alpha pinene, alpha terpineol, alpha thujone, ambrox, ambroxan, amyl acetate, amyl cinnamic aldehyde, amyl salicylate, and isopropyl quinoline, anethol, anisic aldehyde, applinate, aurantiol, bacdanol, benzaldehyde citral polysantol, benzyl acetate, benzyl alcohol, benzyl salicylate, beta gamma hexenol, beta naphthol methyl ether, beta pinene, borneol, bornyl acetate, buccoxime, butyl anthranilate, calone, calone 1951, camphor, carvacrol, carvone,

cashmeran, cashmeran, cedramber, cetalox, cinnamic alcohol, cimnnamic aldehyde, cis 1,3-oxathiane-2-methyl-4-propyl, cis 3 hexenyl acetate, cis-3-hexenyl acetate, cis-3-hexenyl salicylate, cis-6 nonenol, citral, citronellal nitrile, citronellol, citronellyl acetate, citronellyl oxyacetaldehyde, clonal, corps cassis 0.1% tec, coumarin. , cyclal c, cyclemax, cyclo galbanate, cymal, damascene, damascenone, damascone alpha, damascone beta, decyl alcohol, decyl aldehyde, delphone, delta damascone , diacetyl, dihydro iso jasmonate, dihydro myrcenol, dimethyl benzyl carbinol, dimethyl benzyl carbinyl butyrate, dimethyl benzyl carbinyl isobutyrate, d-limonene, ebanol, ethyl 2 4 decadienoate, ethyl 2 methyl butyrate, ethyl aceto acetate, ethyl anthranilate, ethyl butyrate, ethyl caproate, ethyl maltol, ethyl methyl dioxolane acetate, ethyl methyl phenyl glycidate, ethyl vanillin, ethyl-2-4-decadienoate, ethyl-2-methyl- butyrate, eucalyptol, eugenol, exaltolide/cyclopentadecanolide, excital, flor acetate, floralozone, florhydral, fructone, frutene, furaneol, galaxolide, galbex, gamma decalactone, gamma dodecalactone, gamma nonalactone, gamma undecalactone, geraniol, geranyl acetate, geranyl nitrile, geranyl phenylacetate, grapefruit zest (ca), habanolide, helional, heliotropin, heptylcyclopentanone, herbavert, hexahydro-4,7 methano-1h-inden-5(or 6)-yl propionate, hexanoic acid, hexyl acetate, hexyl cinnamic aldehyde, hexyl salicylate, hivemal, hydroquinone dimethyl ether, hydroxycitronellal, indol, intreleven aldehyde, ionone alpha, ionone beta, ionone gamma, irisantheme, iso 2-methoxy-4-(2-propenyl)phenol, isobutyl benzoate, isobutyl cinnamate, iso cyclo citral, iso e super, iso eugenol, iso eugenol acetate, isolongifolanone, iso propyl quinoline, iso-amyl acetate, iso-amyl alcohol, iso methylionone, isononyl acetate, isononyl formate, isovaleric aldehyde 0.1% dpg, javanol, keone, kharismal, labienoxime, lauric aldehyde, lemon juice carbonyls, lilial, liminal, limonene, linalool, linalyl acetate, linalyl benzoate, linalyl cinnamate, lyral, maltol, mandarin aldehyde, manzanate, maple lactone, melonal, menthol, methyl anthranilate, methyl beta naphthyl ketone, methyl cedrylone, methyl cedrylone 0.1041 84.44 811, methyl dihydro jasmonate, methyl dihydrojasmonate, methyl heptine carbonate, methyl isobutenyl tetrahydro pyran, methyl nonyl acetaldehyde, methyl nonyl ketone, methyl octine carbonate, methyl phenyl carbinyl acetate, methyl phenyl carbonyl acetate, methyl-3,4-dioxy(cylcoacetonyl) benzene, nectaryl, neobutanone, nerol, nirvanol, nonalactone, nonanediol-1,3-diacetate, nonanolide-1,4, nonyl aldehyde, nonyl formate, norlimbanol, octyl aldehyde, orange juice carbonyls, orange sinensal, ortho tertiary butyl cyclohexanyl acetate, oxane, oxocyclohexadecen-2-one, ozonil, p.t. bucinal, p-1-menthen-8thiol, para cresol, para cresyl methyl ether, para cymene, para hydroxy phenyl butanone, para hydroxyl phenyl butanone, paradiff, paramenthene, patchouli, pentalide, phenoxy ethyl isobutyrate, phenoxy ethyl propionate, phenyl acetaldehyde, phenyl acetaldehyde dimethyl acetal, phenyl ethyl alcohol, phenyl ethyl dimethyl carbinol, phenyl propyl alcohol, pinoacetaldehyde, polysantol, prenyl acetate, pyrazines, pyrazobutyle, pyridine acetyl 10%, rhubofix, sandalore, sandalore, sandela, spirogalbone, sulfurol, tangerinal, tetra hydro 3,7-dimethyl-1,6-octadien-3-ol, tetrahydrolinalool, tetrahydro muguol, thymol, trans 4 decenal, trans-2-hexenal, tricyclo decenyl acetate, tridecene-2-nitrile, triethyl citrate, trifemal, triplal, undecalactone, undecavertol, undecyl aldehyde, undecylenic aldehyde, vanillin, veloutone, veloutone, verdox, violiff, yara yara, zingerone.

Some of such perfume raw materials have a boiling point of less than, or equal to, 250° C, including some which are generally known to have a low odour detection threshold. Others within said list of perfume raw materials have a boiling point of greater than 250°C of which some are also generally known to have a low odour detection threshold.

Alternatively or additionally, the fragrance incorporated into the capsules can comprise one or a mixture of perfume essential oils, either mixed with each or and/or with synthetic analogues and/or one or more individual perfume compounds, possibly extracted from blossom, leaves, seeds fruit or other plant material. Oils which are herein contemplated include oils from:-

Bergamot, cedar atlas, cedar wood, clove, geranium, guaiacwood, jasmin, lavender, lemongrass, lily of the valley, lime, neroli, musk, orange blossom, patchouli, peach blossom, petotgrain, pimento, rose, rosemary, and thyme,.

It will be recognised that since naturally derived oils comprise a blend in themselves of many components, and the perfume oil commonly comprises a blend of a plurality of synthetic or natural perfume compounds, the perfume oil itself in the encapsulate does not exhibit a single boiling point, ClogP or ODT, even though each individual compound present therein does.

If desired, the composition can include one or more perfume ingredients that provide an additional function beyond smelling attractively. This additional function can comprise deodorancy. Various essential oils and perfume ingredients, for example those passing a deodorant value test as described in US 4278658 provide deodorancy as well as malodour masking.

In the invention described herein, the carrier oil in which the capsules (and the antiperspirant active) are suspended comprises one or more oils, by which is meant liquids that are water-immiscible. Such oils are characterised by being liquid at 20°C (at 1 atmosphere pressure) and are often selected from silicone oils, hydrocarbon oils, ester oils, ether oils and alcohol oils or a mixture of two or more oils selected from such classes of oils. It is highly desirable that the oil has a boiling point of above 100°C and preferably above 150°C.

One class of oils that is highly favoured comprises volatile silicone oils, which often contribute from 20% to 95% by weight of a blend of oils, particularly at least 30% and in many convenient blends at least 40% by weight. It is

advantageous in the instant invention to employ a blend in which the weight proportion of the volatile silicone oils is up to 80% by weight, and particularly up to 70% by weight. The balance of the oils in the blend is provided by one or more non-volatile silicone oils and/or one or more of the other classes of oils.

Herein, a volatile silicone oil is a liquid polyorgano-siloxane having a measurable vapour pressure at 25°C of at least 1 Pa, and typically in a range of from 1 or 10 Pa to 2kPa. Volatile polyorganosiloxanes can be linear or cyclic or mixtures thereof. Preferred cyclic siloxanes, otherwise often referred to as cyclomethicones, include polydimethylsiloxanes and particularly those containing from 3 to 9 silicon atoms, preferably at least 4 and especially at least 5 silicon atoms. Preferred cyclomethicones contain not more than 7 silicon atoms and very preferably up to 6 silicon atoms. Volatile silicone oils herein desirably contain on weight average from 4.5 to 5.9 silicone atoms, and especially at least 4.9.

Preferred linear polyorganosiloxanes include polydimethylsiloxanes containing from 3 to 9 silicon atoms. The volatile siloxanes normally by themselves exhibit viscosities of below $10^{-5}$ $m^2$/sec (10 centistokes), and particularly above $10^{-7}$ $m^2$/sec (0.1 centistokes), the linear siloxanes normally exhibiting a viscosity of below 5 x $10^{-6}$ $m^2$/sec (5 centistokes). The volatile silicones can also comprise linear or cyclic siloxanes such as the aforementioned linear or cyclic siloxanes substituted by one or more pendant -0-$Si(CH_3)_3$ groups, the resultant compounds desirably containing not more than 7 silicon atoms. Examples of commercially available silicone oils include oils having grade designations 344, 345, 244, 245 and 246 from Dow Corning Corporation; Silicone 7207 and Silicone 7158 from Union Carbide Corporation; and SF1202 from General Electric.

Highly desirably, the compositions according to the present invention comprise either an ether oil or an ester oil or both, preferably in a proportion of greater than 10% w/w of the composition, and particularly greater than 20% w/w. Although together they could constitute up to 100% w/w of the carrier oils blend, it is desirable that together they contribute no greater than 60% w/w and in many compositions, they total up to 50% w/w of the blend.

The ester oils can be aliphatic or aromatic. Suitable aliphatic ester oils comprise at least one residue containing from 10 to 26 carbon atoms and a second residue of at least 3 carbon atoms up to 26 carbon atoms. The esters may be mono or diesters, and in the latter be derived from a $C_3$ to $C_8$ diol or di carboxylic acid. Examples of such oils include isopropyl myristate, isopropyl palmitate, myristyl myristate.

It is especially desirable to employ an aromatic ester, including especially benzoate esters. Preferred benzoate esters satisfy the formula Ph-CO-O-R in which R is:-

an aliphatic group containing at least 8 carbons, and particularly from 10 to 20 carbons such as from 12 to 15, including a mixture thereof;

or an aromatic group of formula -A-Y-Ph in which A represents a linear or branched alkylene group containing from 1 to 4 carbons and Y represents an optional oxygen atom or carboxyl group. Particularly preferably, the aromatic ester comprises $C_{12-15}$ alkyl benzoate.

The ether oil preferably comprises a short chain alkyl ether of a polypropylene glygol (PPG), the alkyl group comprising from C2 to C6, and especially C4 and the PPG moiety comprising from 10 to 20 and particularly 14 to 18 propylene glycol units. An especially preferred ether oil bears the INCI name PPG14-butyl ether.

The ester and ether oils herein are selected having a boiling point in excess of 100°C. This enables them to be employed with all wax systems for solidifying the carrier oil that typically melt at no higher than 95°C, and commonly between 65 and 85°C. For sticks made using small molecule gelling agents, it is preferable to select oils having a boiling point in excess of 150°C, and they, naturally, are suitable in conjunction with wax systems too.

The ester and ether oils can be present in the composition in a weight ratio to each other of from 1:0 to 0:1, and in some embodiments from 10:1 to 1:10.

Indeed, though such oils have a number of other beneficial properties, such as for example, reducing the extent to which the antiperspirant formulation is visible after application on the skin, compositions in which the oil blend contains only a minor as compared with a major proportion of such ether and ester oils tend to exhibit sensory attributes preferred by many consumers. In practice, it is desirable for greater than 5% by weight of the oil blend, especially greater than 10% and especially greater than 15% by weight of the oil blend to be furnished by the ester and ether oils. The combined weight of the two oils is preferably less than 60%, particularly less than 50% and especially less than 40% of the weight of the oil blend.

The carrier oil blend can further comprise one or more other water-immiscible oils that have a melting point of below 20°C and a boiling point of above 100°C and preferably above 150°C, including hydrocarbon oils, including preferably non-volatile hydrocarbon oils, non-volatile silicone oils and aliphatic monohydric alcohols.

In the instant invention, non-volatile oils, sometimes referred to as emollient oils, such as non-volatile silicone or/and hydrocarbon oils can desirably be included to alter the sensory attributes of the compositions containing, such as to soften the skin or to assist in masking the visibility of particulate materials deposited on the skin. However, it is desirable to restrict the proportion of such non-volatile oils to less than 30% by weight of the oil blend, and in various compositions according to the instant application, the total proportion of such oils is from 5 to 20% by weight.

Examples of suitable non-volatile hydrocarbon oils include polyisobutene and hydrogenated polydecene. Examples of suitable non-volatile silicone oils include dimethicones and linear alkylarylsiloxanes. The dimethicones typically have an intermediate chain length, such as from 20 to 100 silicon atoms. The alkylarylsiloxanes are particularly those containing from 2 to 4 silicon atoms and at least one phenyl substituent per silicon atom, or at least one diphenylene group. The aliphatic alcohol desirably is a branched chain monohydric alcohol containing from 12 to 40 carbon atoms, and often from 14 to 30 carbon atoms such as isostearyl alcohol.

One further class of ester oils that can constitute a fraction of the ester oils contemplated in the invention compositions comprises natural plant oils, commonly containing glyceride esters and in particular the glyceride triesters of unsaturated C18 aliphatic carboxylic acids, such as linoleic acid, linolenic acid or ricinoleic acid, including isomers such as linolenelaidic acid, trans 7-octadecenoic acid, parinaric acid, pinolenic acid punicic acid, petroselenic acid, columbinic acid and stearidonic acid. Examples of such beneficial natural oils include castor oil, coriander seed oil, impatiens balsimina seed oil, parinarium laurinarium kernel fat, sabastiana brasilinensis seed oil borage seed oil, evening primrose oil, aquilegia vulgaris oil, for and sunflower oil and safflower oil. Such oils can desirably comprise from 1 to 10% by weight of the oil blend.

The weight of fragrance materials is not included herein in calculating the weight of the oil blend, irrespective of whether the fragrance is encapsulated or "free".

Antiperspirant Actives

The composition preferably contains an antiperspirant active. Antiperspirant actives are preferably incorporated in an amount of from 0.5-50%, particularly from 5 to 30% and especially from 10% to 26% of the weight of the composition. It is often considered that the main benefit from incorporating of up to 5% of an antiperspirant active in a stick composition is manifest in reducing body odour, and that as the proportion of antiperspirant active increases, so the efficacy of that composition at controlling perspiration increases.

Antiperspirant actives for use herein are often selected from astringent active salts, including in particular aluminium, zirconium and mixed aluminium/zirconium salts, including both inorganic salts, salts with organic anions and complexes. Preferred astringent salts include aluminium, zirconium and aluminium/zirconium halides and halohydrate salts, such as chlorohydrates.

Aluminium halohydrates are usually defined by the general formula $Al_2(OH)_xQ_y.wH_20$ in which Q represents chlorine, bromine or iodine, x is variable from 2 to 5 and x + y = 6 while $wH_2O$ represents a variable amount of hydration. Especially effective aluminium halohydrate salts, known as activated aluminium chlorohydrates, are described in EP-A-6739 (Unilever NV et al), the contents of which specification is incorporated herein by reference. Such activated aluminium chlorohydrates are made by a method in which the weight concentration of aluminium compounds in the solution is controlled within specified limits and simultaneously the temperature of that solution is controlled within a specified elevated temperature range whilst polymeric aluminium species are formed, and drying conditions are strictly controlled as described in the said EP-A-6739. Some activated salts do not retain their enhanced activity in the presence of water but are useful in substantially anhydrous formulations, i.e. formulations that do not contain a distinct aqueous phase.

Zirconium actives can usually be represented by the empirical general formula: $ZrO(OH)_{2n-nz}B_z.wH_20$ in which z is a variable in the range of from 0.9 to 2.0 so that the value 2n-nz is zero or positive, n is the valency of B, and B is selected from the group consisting of chloride, other halide, sulphamate, sulphate and mixtures thereof. Possible hydration to a variable extent is represented by $wH_20$. Preferable is that B represents chloride and the variable z lies in the range from 1.5 to 1.87. In practice, such zirconium salts are usually not employed by themselves, but as a component of a combined aluminium and zirconium-based antiperspirant.

The above aluminium and zirconium salts may have co-ordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula $CH_2(NH_2)COOH$.

It is highly desirable to employ complexes of a combination of aluminium halohydrates and zirconium chlorohydrates together with amino acids such as glycine, which are disclosed in US-A-3792068 (Luedders et al). Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine. Actives of this preferred type are available from Westwood, from Summit and from Reheis, though with differing particle size distributions.

Many aluminium and/or zirconium-containing astringent antiperspirant salts employed herein have metal: chloride mole ratio in the range of 1.3:1 to 1.5:1. Others having a lower metal:chloride mole ratio, such as from 1:1 to 1.25:1 tend to generate lower pHs when applied to skin and thus tend to be more irritating.

The proportion of solid antiperspirant salt in a suspension composition normally includes the weight of any water of hydration and any complexing agent that may also be present in the solid active.

Many particulate antiperspirants employed in the instant invention have a refractive index (RI) of at least 1.49 and not higher than 1.57. Actives which are free from zirconium tend to have an RI of from 1.49 to 1.54, depending on their formula and at least partly on their residual water content. Likewise, actives which contain zirconium tend to have an RI of from 1.52 to 1.57.

The selection of the antiperspirant active material desirably takes into account the type of applicator from which it is dispensed. Thus, in many particularly preferred embodiments in which the composition is dispensed from a contact applicator, for example using a stick or cream (soft solid dispenser, the antiperspirant active comprises an aluminium-zirconium active, such as AZAG. However, in some other preferred embodiments, the antiperspirant active is highly desirably an aluminium chlorohydrate (ACH) and in others an activated aluminium chlorohydrate (AACH).

For incorporation of compositions according to the present invention, desirably at least 90%, preferably at least 95% and especially at least 99% by weight of the particles have a diameter in the range of from 0.1 $\mu$m up to 100$\mu$m, and usually have an average particle diameter of at least 1 $\mu$m and especially below 20$\mu$m. In some highly desirable contact compositions the particles by weight have a weight average particle size of at least 2$\mu$m and particularly below 10$\mu$m, such as in the range of from 3 to 8$\mu$m.

One method of seeking to minimise visible whiteness employs antiperspirant active material that is free or substantially free from hollow particles. In this context, substantially free indicates a content of less than 10% by weight hollow spheres, and preferably less than 5% by weight. Some drying techniques, eg spray drying, can produce materials which contain greater than such a proportion of hollow spheres, the proportion can be reduced by milling the particulate material, such as by ball or swing milling.

The invention compositions include one or more thickeners or gellants (sometimes called structuring or solidifying agents) to increase the viscosity of or solidify the oil blend in which the particulate materials are suspended as is appropriate for application from respectively soft solid (anhydrous cream) dispensers or stick dispensers. Such thickeners or gellants are selected by the skilled man and enough of them are incorporated to attain the desired viscosity or hardness of the resulting soft solid composition, the actual amount employed taking into account the inherent thickening or gelling capability of the chosen material or combination of materials and their ability to form such a chosen form.

For use as a soft solid, sufficient thickener is introduced to increase the viscosity of the resultant composition to a hardness of from 0.003 to 0.5 Newton/mm$^2$, and commonly from 0.003 or 0.01 up to 0.1 Newton/mm$^2$. Hardness can be measured using a Stable Micro systems TA.XT2i Texture Analyser. A metal sphere, of diameter 9.5mm, is attached to the underside of its 5 kg load cell, and positioned just above the sample surface. Under control of *Expert Exceed*™ software, the sphere is indented into the sample at an indentation speed of 0.05mm/s for a distance of 7mm and reversed to withdraw the sphere from the sample at the same speed. Data comprising time(s), distance (mm) and force (N) is acquired at a rate of 25 Hz. The hardness H at a penetration of 4.76mm is calculated using the formula

$$H = F/A$$

in which H expressed in N.mm$^{-2}$, F is the load at the same travelled distance in N and A is the projected area of the indentation in mm$^{-2}$.

Stick compositions herein desirably have a hardness as measured in a conventional penetration test (Seta) of less than 30mm, preferably less than 20 mm and particularly desirably less than 15 mm. Many have a penetration of from 7 to 13 or 7.5 to 10 or 12.5 mm. The conventional penetration test employed herein, utilises a lab plant penetrometer equipped with a Seta wax needle (weight 2.5 grams) which has a cone angle at the point of the needle specified to be 9□10’ +/- 15’. A sample of the composition with a flat upper surface is used. The needle is lowered onto the surface of the composition and then a penetration hardness measurement is conducted by allowing the needle with its holder to drop under the combined weight of needle and holder of 50 grams for a period of five seconds after which the depth of penetration is noted. Desirably the test is carried out at six points on each sample and the results are averaged.

The gellants for forming stick compositions herein are usually selected from one or more of two classes, viz - non-polymeric fibre-forming gellants sometimes referred to as small molecule gelling agents (viz SMGAs), and waxes, optionally supplemented if desired by incorporation of a particulate silica and/or an oil-soluble polymeric thickener.

The term "wax" is conventionally applied to a variety of materials and mixtures which have similar physical properties, namely that:-

they are solid at 30°C and preferably also at 40°C;

they melt to a mobile liquid at a temperature above 40°C and generally below 95°C and preferably in a temperature range of 55°C to 90°C;

they are water-insoluble and remain water-immiscible when heated above their melting point.

Waxes employed herein as gellants, or in other embodiments as thickeners, are often selected from hydrocarbons, linear fatty alcohols, silicone polymers, esters of fatty acids or mixtures containing such compounds along with a minority (less than 50% w/w and often less than 20% w/w) of other compounds. Naturally occurring waxes are often mixtures of compounds which include a substantial proportion of fatty esters.

Waxes usually form crystals in the water-immiscible liquid when it cools from the heated state during processing, often taking the form of needles or platelets depending on the specific wax.

Examples of hydrocarbon waxes include paraffin wax, ozakerite, microcrystalline wax and polyethylene wax, the last named desirably having an average molecular weight of from 300 to 600 and advantageously from 350 to 525.

Linear fatty alcohols commonly contain from 14 to 40 carbon atoms and often from 16 to 24. In practice, most contain an even number of carbon atoms and many comprise a mixture of compounds, even those that are nominally a single one such as stearyl alcohol. Other alcohols include behenyl alcohol

Examples of ester waxes include esters of $C_{16}$-$C_{22}$ fatty acids with glycerol or ethylene glycol, which can be isolated from natural products or more conveniently synthesised from the respective aliphatic alcohol and carboxylic acid.

Examples of natural waxes include beeswax, woolwax and spermeceti wax of animal origin, and caster wax, jojoba wax, carnauba wax and candelilla wax which are of vegetable origin. The vegetable waxes are commonly obtained by hydrogenation of the corresponding plant oil, containing triglyceride esters of unsaturated fatty acids. Mineral waxes can be extracted from fossil remains other than petroleum. Montan wax, which is an example of mineral wax, includes non-glyceride esters of carboxylic acids, hydrocarbons and other constituents.

Further waxes employable herein comprise silicone polymer waxes, including waxes which satisfy the empirical formula:-

$$R\text{-}(SiMe_2\text{-}O\text{-})_x\text{-}SiMe_2R$$

in which x is at least 10, preferably 10 to 50 and R represents an alkyl group containing at least 20 carbons, preferably 25 to 40 carbons, and particularly having an average linear chain length of at least 30 carbons.

Other silicone waxes comprise copolymers of dimethicone and alkyloxymethicone, satisfying the general formula:-

$$Y\text{- }(SiMe_2\text{-}O\text{-})_y(Si[OR']Me\text{-}O\text{-})_z\text{-}Y'$$

in which Y represents $SiMe_2$-O, Y' $SiMe_2$, R' an alkyl of at least 15 carbons preferably 18 to 22 such as stearyl, y and z are both integers, totalling preferably from 10 to 50.

Some preferred combinations of waxes include stearyl alcohol with an ester wax such as cater wax, often in a weight ratio of from 10:1 to 3:1.

Waxes useful in the present invention will generally be those found to thicken water-immiscible oils such as cyclomethicones when dissolved therein (by heating and cooling) at a concentration of 5 to 15% by weight.

The second class of thickeners or gellants for sticks for soft solids comprises fibre-forming SMGAs. Such gellants are dissolved in a water-immiscible blend of oils at elevated temperature and on cooling precipitate out to form a network of very thin strands that are typically no more than a few molecules wide. One particularly effective category of such thickeners comprises N-acyl aminoacid amides and in particular linear and branched N-acyl glutamic acid dialkylamides, such as in particular N-lauroyl glutamic acid di n-butylamide and N-ethylhexanoyl glutamic acid di n-butylamide and especially mixtures thereof. Such amido gellants can be employed in anhydrous compositions according to the present invention, if desired, with 12-hydroxystearic acid.

Other amido SMGAs include 12-hydroxystearic acid amides, and

amide derivatives of di and tribasic carboxylic acids as set forth in WO 98/27954, including notably alkyl N,N'dialkyl succinamides

Further suitable amido-containing SMGAs are described in US6410003 and satisfy the general formula:-

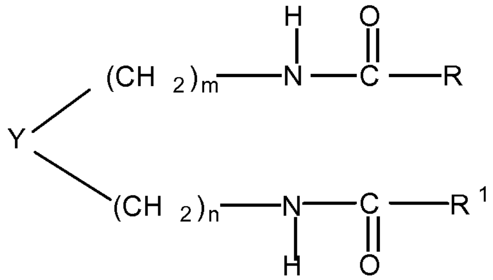

in which m and n are each independently 1 or 0, and Y represents a cyclohexane group substituted in the ortho or para positions by the respectively amido substituents in which R and R' each represents an aliphatic group containing from 5 to 25 carbons.

A class of amido-containing SMGAs including some specially efficient gellants comprises cyclodipeptide derivatives disclosed in US7332153 having the general formula

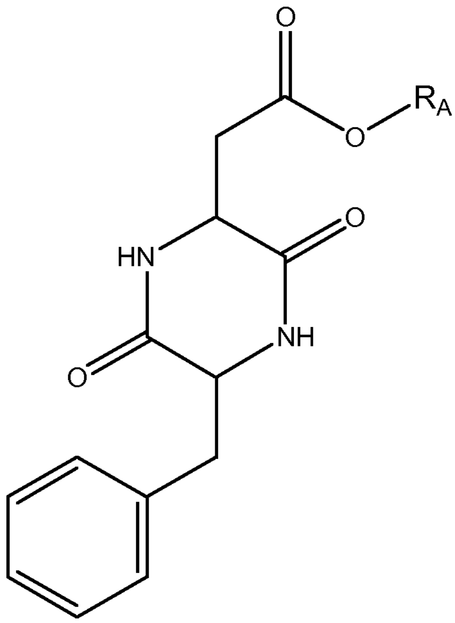

in which $R_A$ represents a carbocyclic or heterocyclic group containing not more than 2 rings, other than unsubstituted cyclohexyl. Preferred compounds include such cyclodipeptide derivatives in which the residue $R_A$ is derivable from thymol, isopinocamphenol, a 3,5-dialkyl cyclohexanol, carveol.or carvacrol.

A further class of suitable SMGAs is described in US6410001 and satisfies the general formula

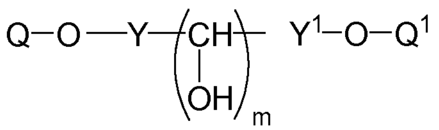

in which in which Y and $Y^1$ are each independently $-CH_2-$ or >CO, Q and $Q^1$ are each independently a hydrocarbyl group of at least 6 carbon atoms, and m is from 2 to 24, and particularly gellants in which the substituents terminate in a cyclohexyl ring, optionally substituted by C1, Br, F, OH, $NO_2$, $O-CH_3$, or $CH_3$.

A combination SMGA which is also suitable which is described in US6321841 is a combination of a sterol and a sterol ester and in particular β-sitosterol together with γ-oryzanol preferably in the range of from 3:1 to 1:2.

A further class of SMGAs as described in US6248312 comprises esters of cellobiose and a fatty acid, preferably of 6 to 13 carbon atoms especially 8 to 10 carbon atoms and particularly nonanoic acid. Especially desirably, the cellobiose is esterified, on average, by between 7 and 8 groups, and most desirably adopts the α-anomeric form.

Naturally, a combination of two or more gellants can be employed, such as a wax or mixture of waxes alone, or a mixture of SMGAs alone of a mixture of a wax or waxes plus an SMGA or SMGAs, such as are described hereinabove.

The gellant is often employed in the stick or soft solid composition at a concentration of from 1.5 to 30%, depending on the nature of the gellant or gellants, the constitution of the oil blend and the extent of hardness desired. When an SMGA is employed as the principal gellant, its concentration is typically in the range of from 1.5 to 7.5% w/w for amido gellants or mixtures of them and for 5 to 15% for ester or sterol gellants. When a wax is employed as the principal gellant, its concentration is usually selected in the range of from 10 to 30% w/w, and particularly from 12 to 24% w/w.

If a wax is used which forms a network of fibres, the amount of it may be from 0.5 to 7% by weight of the composition. If a wax is used which does not form such a network, for instance a wax which crystallizes as spherulitic needles or as small platelets, the amount may well be from 2% or 3% up to 10%, 12% or 15% of the composition. Silicone waxes are an example of waxes which crystallize as small platelets.

Some highly desirable compositions comprise in combination a first gellant eg wax with a second gellant, eg SMGA. The total amount of second gellant may range from 0.5% or 1% of the composition up to 9%, 10% or 15%.

In general, soft solid compositions herein can include one or more of the gellants employed to make a firm stick as described above, but employing a lower concentration of the respective gellant. Thus, the concentration of such gellants is often selected in the range of from 0.5 to 15% w/w of the composition and in many instances from 1 to 10% w/w.

However, it can be especially desirable to employ an oil-soluble polymer as thickening agent for forming a soft solid, for example selected in the range of from 2 to 20% w/w of the composition. Likewise such polymers can be included in stick compositions.

One category of oil-soluble polymer which has been found suitable is a polysaccharide esterified with monocarboxylic acid containing at least 12 carbon atoms, and preferably a dextrin fatty acid ester such as dextrin palmitate or dextrin stearate. Commercial products are available under the trade mark Rheopearl.

A second category of polymer thickener comprises polyamides for example those discussed in US 5500209. Such polyamides may be derived from organic diamines containing 2 to 12, preferably 2 to 8 carbon atoms, condensed with di- or poly carboxylic acids containing 4 to 20 carbon atoms per carboxylic acid group, for example VERSAMID 950 derived from hexamethylene diamine and adipic acid. Further polyamides that are copolymers with polysiloxanes are described in US 6353076, and particularly its copolymers of Formulae I, II, III, or IV, or as prepared n any of Examples 1 to 5 therein.

A third category of thickening comprises block copolymers of styrene with ethylene propylene and/or butylene available under the trade name KRATON, and particularly styrene ethylene/butylene styrene linear block copolymers. A related category of thickening polymer comprises polymers of alpha methylstyrene and styrene, such as those under the trade name KRISTALEX, eg KRISTALEX F85 having a mean molecular weight of approximately 1200. Yet another thickening polymer comprises alkyl substituted galactomannan available under the trade name N-HANCE AG.

A still further class of thickening polymers co-polymers of vinyl pyrrolidone with polyethylene containing at least 25 methylene units, such as triacontanyl polyvinylpyrrolidone, under the trade name Antaron WP-660.

Such thickening polymer is often employed in a weight ratio to the oil blend that is selected in the range of from 1:30 to 1:5, taking into account the hardness of the soft solid that is desired, the inherent ability of the chosen polymer to increase viscosity and the presence or otherwise of an additional thickener.

A further class of material which is well suited to forming of contributing to the formation of soft solid compositions comprises silicone elastomers. Such materials are conventionally formed by the hydrosilation of vinyl silicone fluids by hydrosiloxane or MQ hydride fluids. Commonly, for anhydrous compositions, the elastomer is non-emulsifying and especially is a dimethicone/vinyldimethicone cross polymer. Such materials are capable of absorbing a substantial proportion of hydrophobic oils, including cyclomethicones, and are commonly supplied as a dispersion of the active material in cyclomethicone fluid or a non-volatile oil, typically at a concentration in the region of 10 to 20% by weight. Such elastomers are desirably present at a concentration of from 1 to 10% by weight of the composition.

A thickener especially well suited to forming or contributing to the formation of a soft solid composition comprises a particulate silica and especially a fumed silica. It is desirable to include at least 2% and especially at least 2.5% by weight of the silica in the composition, such as in the range of up to 10% by weight.

The anhydrous compositions can contain one or more optional ingredients, such as one or more of those selected from those identified below.

Optional ingredients include wash-off agents, often present in an amount of up to 10% w/w to assist in the removal of the formulation from skin or clothing. Such wash-off agents are typically nonionic surfactants such as esters or ethers containing a $C_8$ to $C_{22}$ alkyl moiety and a hydrophilic moiety which can comprise a polyoxyalkylene group (POE or POP) and/or a polyol.

The compositions herein can incorporate one or more cosmetic adjuncts conventionally contemplatable for cosmetic solids or soft solids. Such cosmetic adjuncts can include skin feel improvers, such as talc or finely divided (i.e. high molecular weight) polyethylene, i.e. not a wax, for example Accumist™, in an amount of 1 up to about 10%; a moisturiser, such as glycerol or polyethylene glycol (mol wt 200 to 600), for example in an amount of up to about 5%; skin benefit agents such as allantoin or lipids, for example in an amount of up to 5%; colours; skin cooling agents other

than the already mentioned alcohols, such a menthol and menthol derivatives, often in an amount of up to 2%, all of these percentages being by weight of the composition. A further optional ingredient comprises a preservative, such as ethyl or methyl paraben or BHT (butyl hydroxy toluene) such as in an amount of from 0.01 to 0.1% w/w.

The invention compositions herein can additionally contain a water-soluble polymer comprising a Bronsted acid group that cooperates synergistically with the aluminium or aluminium/zirconium antiperspirant active to enhance antiperspirant efficacy. Such a material is referred to in US6616921 as a co-gellant (because it assists the antiperspirant active to gel in eccrine pores) and is described therein. Preferred examples of such a co-gellant are polymers having a molecular weight of at least 50,000 derived at least in part from maleic acid or maleic anhydride, such as Gantraz™ AN119, AN139 or AN169. The co-gellant is often selected in a weight ratio to the aluminium or aluminium/zirconium salt of from 1:15 to 1:2.

The invention compositions herein can additionally comprise a non-encapsulated fragrance, for example in a weight % of from 0.01 to 4% of the composition, and particularly from 0.1 to 1.5%. The non-encapsulated fragrance can be created from the same palette of perfume materials described above. The non-encapsulated fragrance can, if desired, be the same as or similar to the encapsulated fragrance, but it is often more attractive if the two fragrances are different, because this minimises the extent to which the nose has become desensitised to perfume. Choice of the various fragrances and the differences between them, such as proportion of top notes, is primarily a matter of aesthetic judgement.

Additionally or alternatively to the non-encapsulated fragrance, if desired the compositions herein can comprise fragrance encapsulated in a water-sensitive shell, such that when a person sweats, the aqueous excretion ruptures the shell releasing fragrance. Such water-sensitive encapsulates are described for example in EP0303461. Additionally or likewise alternatively, the compositions herein can comprise a cyclic oligosaccharide such as cyclodextrins, including $\alpha$ or $\beta$ cyclodextrin, each optionally substituted by a methyl or hydroxy-propyl group that associates reversibly with free fragrance. Such materials are described in EP1289484.

The invention compositions desirably are substantially or totally free from water-soluble short chain monohydric alcohols (commonly recognised as up to $C_6$) and especially ethanol. Substantially in this context indicates a proportion of less than 5% and preferably less than 1% by weight of the respective full or base composition.

Herein unless the context demands otherwise, all weights, %s, and other numbers can be qualified by the term "about".

Preparation of stick and soft solid compositions:

The invention compositions can be made by way of the methods hitherto described for making respectively anhydrous stick or soft solid compositions containing an antiperspirant or deodorant active ingredient. However, it is especially desirable for the fragrance capsules to be incorporated into the composition with gentle mixing, at a rate and power input that does not damage the capsules, and, for the same reason, the composition is subsequently subjected to shear or equivalent intensive mixing such as by employing a bladed high shear mixer, eg a Silverson™ mixer or by passage through an in-line mixer such as a Sonolator™.mixer.

One convenient process sequence for preparing a stick or soft composition according to the present invention comprises first forming a solution of the structurant combination in the water-immiscible liquid or one of the water-immiscible liquids. This is normally carried out by agitating the mixture at a temperature sufficiently high that all the structurants dissolve (the dissolution temperature) such as a temperature in a range from 70 to 140°C. Any oil-soluble cosmetic adjunct can be introduced into oil phase, either before or after the introduction of the gellants. However, the fragrance oil, be it encapsulated or free, is commonly the last ingredient to be incorporated into the composition, after the antiperspirant active on account of its sensitivity often to elevated temperature. Commonly the resultant structurant solution is allowed to cool to a temperature that is intermediate between that at which the gellants dissolved and the temperature at which it would set, often reaching a temperature in the region of 60 to 90°C.

In some routes, the carrier oils can be mixed together prior to introduction of the gellants and the antiperspirant or deodorant active. In other preparative routes, it is desirable to dissolve all or a fraction of the gellants and especially for amido gellants in a first fraction of the composition, such as a branched aliphatic alcohol, e.g. isostearyl alcohol or octyldodecanol, optionally in conjunction with an alcohol having some water-miscibility and boiling point above the dissolution temperature of the amido gellant in the alcoholic fluid. This enables the remainder of the carrier fluids to avoid being heated to the temperature at which the structurants dissolve or melt. Such a process commonly involves mixing the fractions intensively in for example a "Sonolator"™. In the invention compositions, the fragrance capsules are most desirably introduced after any intensive mixing step. The proportion of the carrier fluids for dissolving the structurants is often from 25 to 50% by weight of the carrier fluids.

In said other preparative routes the particulate material is introduced into preferably a second fraction of the carrier oils, for example silicone and/or ester and/or hydrocarbon oils and thereafter, and thereafter the first fraction containing dissolved structurant and second fraction containing suspended particulate material are mixed at a temperature above that at which the composition gels, and often from 5°C to 30°C above the regular setting temperature of

the composition, dispensing containers are filled and cooled or allowed to cool to ambient temperature. Cooling may be brought about by nothing more than allowing the container and contents to cool. Cooling may be assisted by blowing ambient or even refrigerated air over the containers and their contents.

Product Dispenser

Although the stick composition could be formed into an extruded bar and wrapped and sold in that form, generally it is desirable to house the composition in a stick dispenser that conventionally comprises a barrel one at one end, and a platform located beneath the open end adapted to propel the stick composition out of the barrel through the open end. The means for propulsion can comprise an opening at a second end of the barrel remote from the first end through which a finger could be inserted to come into contact with the underside of the platform, or more usually is rotor wheel at the base of a barrel on which is mounted a threaded spindle that extends through a correspondingly threaded aperture in the platform. The barrel engages the platform so as to prevent rotation of the latter, so that when the rotor wheel and spindle are rotated, the platform is advanced or retracted to or from the open end. Examples of suitable dispensers are described, for example, in US 4232977, US4605330, WO09818695, WO09603899, WO09405180, WO09325113, WO09305678, EP1040445, US5997202, US5897263, US5496122, US5275496, US 6598767, US 6299369, or WO 2002/03830. The dispensers or moulds into which the stick composition is introduced can be made from thermoplastic material, such as polyethylene and commonly contain from 10 to 100g composition, such as in the range of from 15 to 25g for small or sample sticks and from 40 to 80g for regular or large sticks.

Dispensers for soft solids are generally similar to those for firm sticks, except that mostly, the open end of the barrel is fitted with a top wall, often domed, defined at least opne and typically a series of narrow aperture through which the soft solid can be extruded by gentle pressure. The same type of advance mechanism can be employed as for firm sticks. Suitable dispensers for soft or semi-solids are exemplified in US4865231, US5000356, US6116803, US5961007, WO9851185, EP0312165, WO0019860, EP0709041, EP858271, US5573341, US5725133, US5248213, US6398439 or US6450716.

Commonly, the dispenser for sticks or soft solids is moulded from a thermoplastic such as polypropylene or polyethylene.

Having summarised the invention and described it in more detail, together with preferences, specific embodiments will now be described more fully by way of example only.

The dried aminoplast capsules employed in Example 1 herein were made in accordance with the general process described hereinbefore for making capsules having an aminoplast resin shell, employing melamine and formaldehyde under aqueous alkaline conditions to form the precondensate that is mixed under high shear agitation with a selected fragrance oil to form a dispersion of oil droplets having an average diameter of about 40 μm that is thereafter subject to acid catalysis to form a hard shell having the characteristics identified in Table 1 below after drying.

Table 1

| Characteristic | Capsules |
|---|---|
| Mean particle size D[4,3] | 53.1 μm |
| Shell thickness (of 11 to 18μm capsules) | 1.7-4.3 μm |
| Shell thickness calculated at mean particle size | 5.2-7.9 μm |
| DR (of 11 to 18μm capsules) | 4.0:1 - 6.3:1 |
| DR (calculated shell thickness) | 6.7:1 - 10:1 |
| 36:1 Hysitron hardness | 3.07 MPa |
| Apparent Reduced Elastic Modulus | 21.2 MPa |
| Wt % fragrance in core | 45 |

Mean Particle Size: D[4,3] of the capsules after dispersion in volatile silicone (cyclopentadimethicone) was obtained using a Malvern Mastersizer 2000, the following parameters.

- RI of Dispersant = 1.397
- RI of capsules = 1.530

- Dispersion module mixer speed = 2100rpm.
- Result calculation model = General purpose.
- Calculation sensitivity = Normal.
- Particle shape = Spherical

Shell Thickness: Measured by SEM on encaps with a particle size specified. For non-spherical encaps, the thickness was measured at or close to the minimum encapsulate diameter.
Shell Thickness (Calculated): Calculation assumed that capsules were spherical, with a single core and the shell and core had the same density.
DR is the ratio of av. particle diameter: measured shell thickness.

Example 1

In this Example, the effectiveness of the encapsulated fragrance product was compared in the same anhydrous antiperspirant composition with a non-encapsulated fragrance having similar fragrance characteristics.
The effectiveness was determined in the following test in which 24 - 26 panelists self-applied approximately 0.3g example stick product to either the left or right armpit and comparison product to the other, with overall left-right randomized balance. The test formulation containing the encapsulated fragrance plus a fruity-floral (Bm) non-encapsulated fragrance was compared with a control formulation that contained the fruity-floral (Bm) non-encapsulated fragrance alone. The formulations are summarized in Table 2.
After application of the antiperspirant formulations, the users put on their normal clothing and the intensity of the odour was assessed at 2 hourly intervals on a scale of perception increasing from 0 to 10. The scores were averaged and that for the non-encapsulated sample deducted from that for the encapsulated sample. Three scores were measured, namely intensity of the fragrance itself, the intensity detected through the clothing and finally the intensity of any malodour. The results using a representative stick formulation are summarized in Table 3 below.
The tested formulations were as follows:-

Table 2

| | Stick | |
|---|---|---|
| Ingredient | % by weight | |
| | Ex 1 | Control |
| Cyclomethicone | Balance | Balance |
| Ester oil | 15.0 | 15.0 |
| Ether Oil | 9.5 | 9.5 |
| Stearyl alcohol | 18.0 | 18.0 |
| Castorwax | 3.5 | 3.5 |
| Polyethylene wax | 1.0 | 1.0 |
| AZAG | 24.00 | 24.00 |
| Preservative | 0.05 | 0.05 |
| Aminoplast Encapsulated Fragrance | 1.5 | |
| Non-encapsulated Fragrance | 1.5 | 1.5 |

In the stick compositions, the proportion of fragrance employed incorporated within the capsules of Ex 1 was about 0.68% in the stick and the proportion of the non-encapsulated fragrance was substantially higher at the outset of the comparisons.

Table 3 Stick Results (Example 1)

| Assessment time (Hrs) | Difference in Intensity at assessment | | |
|---|---|---|---|
| | Direct | Through Clothing | Malodour |
| 0 | 0.42 | 0.5 | nd |
| 2 | 0.85 | 0.81 | 0 |
| 4 | 0.8 | 0.61 | 0 |
| 6 | 0.5 | 0.3 | -0.2 |
| 8 | 0.38 | 0.38 | -0.12 |
| 10 | 0.54 | 0.31 | -0.27 |
| 12 | 0.2 | 0.5 | -0.35 |
| 14 | 0.23 | 0.34 | -0.12 |

From Table 3, it is apparent that a greater intensity of the fragrance was perceived from the encapsulated fragrance compared with the non-encapsulated alternative throughout the period of the trial, irrespective of whether was assessed through clothing or directly, i.e. not through clothing, and even though a substantially greater amount of the non-encapsulated fragrances were employed. In addition, when judging the presence of malodours, the panelists consistently generated negative differences, once a long enough period had elapsed for malodours to be generated in situ, showing once again the effectiveness of the encapsulated sample at masking malodour. The time span of the test represented a substantial fraction of the waking hours for many consumers.

Example 2

Clinical trials were conducted to demonstrate the difference in malodour suppression between encapsulated (test) and non-encapsulated (control) fragrance applied from a stick composition. The formulations employed in Example 2 were the same as those employed in Example 1. In addition, comparisons were made against the same formulation except for containing a different non-encapsulated fragrance, referred to as Cn.

In this Example, test and control product was applied daily to the underarm of panelists (0.3g +/- 0.03g) and the panelist carried out normal daily activities until after 5 hours, the effectiveness of the fragrance was assessed by the trained assessor rubbing the underarm gently with latex-gloved fingers (10 strokes). After 2 minutes, the malodour was assessed, but on a scale of from 0 to 5. This was repeated on 4 days, with the panelist instructed not to wash the underarm or apply any other antiperspirant or deodorant during the trial.

Table 4 Stick

| Encapsulated Fragrance | Assessment before or after shear | Assessment after application (hours) | Odour Score |
|---|---|---|---|
| Cn | Before | 5 | 0 |
| Cn | After | 5 | -0.18 |
| Bm | Before | 5 | -0.19 |
| Bm | After | 5 | -0.12 |

In Table 4, results with a -ve prefix show a reduction in malodour score relative to the control.

The results summarised in Table 4 show that users assessed that the composition employing the encapsulated fragrance reduced malodour to a greater extent than the compositions containing only non-encapsulated fragrances over the measurement time of 5 hours after application.

Other anhydrous formulations containing an encapsulated fragrance according to the instant invention.
Ingredients for the preparation of the products employed in Examples 1 to 10 respectively.

Table 5

| Ingredient | Name or Trade Name | Supplier |
|---|---|---|
| Cyclomethicone [1] | DC 245 | Dow Corning Inc |

(continued)

| Ingredient | Name or Trade Name | Supplier |
|---|---|---|
| Ester oil 1 [2] | C12-15 alkyl benzoate Finsolv TN | Finetex |
| Ester oil 2 [3] | Isopropyl myristate Estol 1512 | Uniqema |
| Ester Oil 3 | 2-phenyl ethyl benzoate Finsolv SUN | Finetex |
| Ether Oil | INCI PPG-14-butyl ether Fluid AP | Ucon Inc |
| Dimethicone | Dow Corning Fluid 200 (350 cSt) | Dow Corning Inc |
| Branched alcohol [4] | Isostearyl alcohol Prisorine 3515 | Uniqema |
| Stearyl alcohol [5] | Lorol 18 | Cognis |
| Ester wax 1 [6] | Castor wax Castorwax MP80 | CasChem Inc |
| Ester wax 2 [7] | Alkyl stearate behenate Kester Wax 82N | Koster Keunen |
| Ester wax 3 | Triglyceride wax Synchrowax HGL-C | Croda Ltd |
| Hydrocarbon wax 1 | Polyethylene Performalene 400 | New Phase Technologies (Baker Petrolite) |
| Hydrocarbon wax 2 | Paraffin wax SP173P, | Strahl & Pitsch Inc |
| Hydrocarbon Polymer | Styrene-ethylene/ butylene-styrene Block Copolymer Kraton G1650E | Kraton Polymers |
| SMGA 1 | N-(2-ethyl hexanoyl)-L-glutamic acid di-n-butylamide GA-01 | Ajinomoto |
| SMGA 2 | N-lauroyl-L-glutamic acid di-n-butylamide GP1 | Ajinomoto |
| SMGA 3 | 12-hydroxystearic acid | CasChem |
| Silicone Elastomer | 10% w/w in cyclomethicone DC9040 | Dow Corning Inc |
| Fumed silica | fumed silica Cab-o-sil | Cabot |
| AZAG | Aluminium zirconium tatrachlorohydrex-Gly Reach 908 | Reheis Inc |
| Preservative | Butylhydroxytoluene Tenox BHT | Eastman Chemicals |
| Aminoplast encap | As described in Table 1 | IFF |
| Starch Encap | Starch encapsulate | IFF |
| Free Fragrance | Ex Fragrance House | |

Foot notes
[1] DC245 can be replaced wholly or partly by DC246, or DC345™
[2] Finsoln TN can be replaced wholly or partly by Finsolv TPP™
[3] Estol 1512 can be replaced wholly or partly by Estol 1517™
[4] Prisorine 3515 can be replaced wholly or partly by Eutanol G16™, (Cognis)
[5] Lorol 18 can be replaced partly (up to 50%) by Lanette 16™ and/or Lanette 22™
[6] Castorwax MP80 can be replaced wholly or partly by Castorwax MP90™.
[7] Kester Wax 62 can be replaced wholly or partly by Kester Wax 69H

Examples 1 to 8

In these Examples, stick products are made by filling a dispenser comprising a barrel oval in cross section having a base and an open top covered by a cap, a platform fitting snugly within the barrel at a position intermediate between the base and the top and advancement means for the platform mounted under the base, said means comprising a rotor wheel and an attached threaded spindle engaging a cooperating thread in the platform with a composition summarised in the Table below. The summarised stick compositions are made by the following general method.

The selected oil or oils are charged in the desired weight proportion into a vessel, the desired gellant or mixture of gellants in the desired weight proportion is introduced and the resultant mixture is agitated with an agitator of suitable

power or by circulation through a recirculation loop, and heated until a temperature is reached at which the gellant or all the gellants have dissolved in the oils. For waxes that temperature is commonly in the range of from 75 to 90°C. For SMGAs, depending on the particular SMGA, that temperature is often from 90 to 120°C. Thereafter, the mixture is allowed to cool by 5 to 15°C and the desired weight proportion of particulates other than the encapsulated fragrance (including particularly the antiperspirant active) are introduced with continued agitation. The mixture is cooled or allowed to cool to a temperature of about 5 to 10°C above the normal setting temperature of the composition (which has been determined in a previous trial). Finally, with gentle agitation, the encapsulated fragrance and any non-encapsulated (free) fragrance is introduced and the still mobile composition is charged into the dispenser.

Examples 9 and 10

In Examples 9 and 10, soft solid formulations are made. The soft solid formulations are charged into a dispenser having its top covered by a dome with narrow apertures. That made with a wax gellant are made by a similar process to that of the stick formulations, the amount being insufficient to produce a hard stick.

That made using a silica thickening agent comprises stirring a suspension of all the ingredients in a vessel at a temperature in the range of 25 to 50°C until an homogeneous suspension is obtained, and thereafter top filling it into the dispenser and placing the dome in the mouth.

Stick formulations

Table 8

| Example No | 3 | 4 | 5 | 6 | 7 | 8 | 9 | 10 |
|---|---|---|---|---|---|---|---|---|
| Ingredient | | | | | | | | |
| Cyclomethicone | 34.0 | 17.5 | 47.5 | 25.0 | | 37.5 | 32.5 | 32.0 |
| Ester oil 1 | 6.0 | 15.0 | | 17.5 | | 10.0 | 14.0 | 10.0 |
| Ester oil 2 | 6.0 | | | | | | 7.5 | |
| Ester Oil 3 | | | | | 53.15 | | | |
| Ether Oil | 10.0 | 18.0 | 15.0 | 15.5 | | 5.0 | | 5.0 |
| Dimethicone | | | 5.0 | 1.0 | | | 8.0 | 7.0 |
| Branched alcohol | | | | | 11.45 | 14.0 | | |
| Stearyl alcohol | 15.5 | 18.0 | | | | | | |
| Ester wax 1 | 4.0 | 3.5 | | | | | | |
| Ester wax 2 | | | 10.0 | | | | | |
| Ester wax 3 | | | | | | | 3.25 | |
| Hydrocarbon wax 1 | | 1.0 | | 8.0 | | | | |
| Hydrocarbon wax 2 | | | | 6.0 | | | 3.25 | |
| Hydrocarbon Polymer | | | | | 5.9 | | | |
| Silicone elastomer | | | | | | | 4.0 | |
| Fumed Silica | | | | | | | | 5.0 |
| SMGA 1 | | | | | 2.5 | | | |
| SMGA 2 | | | | | 2.5 | 2.5 | | |
| SMGA 3 | | | | | | 7.0 | | |
| ACH | 24.0 | | 20.0 | | | 22.0 | | 12.0 |
| AZAG | | 24.0 | | 24.5 | 22.5 | | 25.0 | 12.0 |
| Aminoplast Encap | 0.5 | 1.5 | 1.5 | 2.0 | 1.0 | 2.0 | 1.5 | 1.5 |

(continued)

| Example No | 3 | 4 | 5 | 6 | 7 | 8 | 9 | 10 |
|---|---|---|---|---|---|---|---|---|
| Starch Encap | | | | 0.5 | | | | 0.5 |
| Free Fragrance | | 1.5 | 1.0 | | 1.5 | | 0.5 | |

## Claims

1. An anhydrous contact antiperspirant composition comprising
a particulate antiperspirant active,
from 0.5 to 2.5% by weight of a dry particulate shear sensitive encapsulated perfume,
a liquid carrier for the particulate antiperspirant active and shear sensitive encapsulated perfume comprising at feast one water-immiscible oil and optionally a thickener or gellant for the oil or a suspension aid or a propellant
in which the particulate encapsulated perfume comprises capsules having a water content of less than 5%,
having an aminoplast shell,
a weight average particle diameter in the range of 25 to 60 μm,
a shell having an average measured thickness in the range of from 2.5 to 8 μm
a ratio of the shell thickness to the average particle diameter in the range of from 1:3 to 1:20
and a Hysitron hardness, measured in a Hysitron Tribo-indenter fitted with a Berkovich tip and programmed to perform an indent by compressing a sample with an initial contact force of 75 μN, for 10 seconds, followed by a position hold stage for 1 second and a decompression stage for 10 seconds, in the range of from 1.5 MPa to 50 MPa.

2. A composition according to claim 1 in which the aminoplast is derived from melamine.

3. A composition according to either preceding claim in which the aminoplast is derived from formaldehyde.

4. A composition according to any preceding claim in which the capsules have an average shell thickness in the range of from 4 to 8μm.

5. A composition according to either preceding claim in which the capsules have an average particle size:shell thickness ratio in the range of from 5:1 to 10:1.

6. A composition according to claim 5 in which the average particle size:shell thickness ratio in the range of from 6.5:1 to 9:1.

7. A composition according to any preceding claim in which the capsules have an average core volume of from 35 to 55% by volume.

8. A composition according to any preceding claim in which the capsules have a Hysitron hardness in the range of from 2.5 to 4MPa.

9. A composition according to any preceding claim which contains from 0.1 to 4% by weight of the capsules.

10. A composition according to any preceding claim which additionally contains non-encapsulated fragrance.

11. A composition according to any preceding claim in which the blend comprises from 30 to 70% w/w volatile silicone oil and from 20 to 40% w/w ester oil and/or ether oil.

12. A composition according to claim 11 in which at least 90% w/w of the volatile silicone oil is cyclopentadimethicone and/or cyclohexadimethicone.

13. A composition according to claim 11 in which the ester oil is an alkyl benzoate and optionally a triglyceride oil of an unsaturated $C_{18}$ fatty acid.

14. A composition according to any preceding claim containing at least one gellant selected from waxes, oil-soluble polymers and non-polymeric fibre-forming gellants at a weight concentration in the composition selected in the range

of from 1.5 to 30% and the composition having a penetration hardness of from 7 to 13mm.

**Patentansprüche**

1. Wasserfreie schweißhemmende Kontaktzusammensetzung, umfassend
einen partikelförmigen, schweißhemmenden Wirkstoff,
von 0,5 bis 2,5 Gewichts-% eines trockenen, partikelförmigen, scherempfindlichen eingekapselten Duftstoffs,
einen flüssigen Träger für den partikelförmigen schweißhemmenden Wirkstoff und scherempfindlichen eingekapselten Duftstoff, umfassend mindestens ein mit Wasser nicht mischbares Öl und optional ein Verdickungsmittel oder Geliermittel für das Öl oder ein Suspendierhilfsmittel oder ein Treibmittel, worin der partikelförmige eingekapselte Duftstoff Kapseln umfasst mit einem Wassergehalt von weniger als 5%,
einer Aminoplast-Schale,
einem gewichtsgemittelten Partikeldurchmesser in dem Bereich von 25 bis 60 μm,
einer Schale einer gemessenen Durchschnittsstärke in dem Bereich von 2,5 bis 8 μm,
einem Verhältnis der Schalenstärke zu dem durchschnittlichen Partikeldurchmesser in dem Bereich von 1:3 bis 1:20 und einer Hysitron-Härte, gemessen in einem Hysitron-Triboindenter, ausgestattet mit einer Berkovich-Spitze und programmiert, um einen Einschnitt durch Komprimieren einer Probe mit einer anfänglichen Kontaktkraft von 75 μN, für 10 Sekunden, gefolgt von einer Positionshaltestufe für 1 Sekunde und einer Dekompressionsstufe für 10 Sekunden auszuführen, in dem Bereich von 1,5 MPa bis 50 MPa.

2. Zusammensetzung nach Anspruch 1, in welcher der Aminoplast von Melamin stammt.

3. Zusammensetzung nach irgendeinem vorhergehenden Anspruch, in welcher der Aminoplast von Formaldehyd stammt.

4. Zusammensetzung nach irgendeinem vorhergehenden Anspruch, in welcher die Kapseln eine durchschnittliche Schalenstärke in dem Bereich von 4 bis 8 μm aufweisen.

5. Zusammensetzung nach irgendeinem vorhergehenden Anspruch, in welcher die Kapseln ein Verhältnis der durchschnittlichen Partikelgröße:Schalenstärke in dem Bereich von 5:1 bis 10:1 aufweisen.

6. Zusammensetzung nach Anspruch 5, in welcher das Verhältnis der durchschnittlichen Partikelgröße:Schalenstärke in dem Bereich von 6,5:1 bis 9:1 liegt.

7. Zusammensetzung nach irgendeinem vorhergehenden Anspruch, in welcher die Kapseln ein durchschnittliches Kernvolumen von 35 bis 55 Volumen-% aufweisen.

8. Zusammensetzung nach irgendeinem vorhergehenden Anspruch, in welcher die Kapseln eine Hysitron-Härte in dem Bereich von 2,5 bis 4 MPa aufweisen.

9. Zusammensetzung nach irgendeinem vorhergehenden Anspruch, welche 0,1 bis 4 Gewichts-% der Kapseln enthält.

10. Zusammensetzung nach irgendeinem vorhergehenden Anspruch, welche zusätzlich nicht-eingekapselten Duftstoff enthält.

11. Zusammensetzung nach irgendeinem vorhergehenden Anspruch, in welcher die Mischung von 30 bis 70% Gew./Gew. flüchtiges Silikonöl und von 20 bis 40% Gew./Gew. Esteröl und/oder Etheröl umfasst.

12. Zusammensetzung nach Anspruch 11, in welcher mindestens 90% Gew./Gew. des flüchtigen Silkonöls Cyclopentadimethicon und/oder Cyclohexadimethicon ist.

13. Zusammensetzung nach Anspruch 11, in welcher das Esteröl ein Alkylbenzoat und optional ein Triglyceridöl einer ungesättigten $C_{18}$-Fettsäure ist.

14. Zusammensetzung nach irgendeinem vorhergehenden Anspruch, die mindestens ein Geliermittel enthält, ausgewählt aus Wachsen, öllöslichen Polymeren und nicht-polymeren faserbildenden Geliermitteln in einer Gewichtskonzentration in der Zusammensetzung, ausgewählt in dem Bereich von 1,5 bis 30%, und wobei die Zusammensetzung

eine Penetrationshärte von 7 bis 13 mm aufweist.

**Revendications**

1. Composition d'antiperspirant à contact anhydre comprenant
une matière active d'antiperspirant particulaire,
de 0,5 à 2,5 % en masse d'un parfum encapsulé sensible au cisaillement particulaire sec,
un support liquide pour la matière active d'antiperspirant particulaire et le parfum encapsulé sensible au cisaillement comprenant au moins une huile immiscible à l'eau et éventuellement un épaississant ou gélifiant pour l'huile ou un auxiliaire de suspension ou un propulseur
dans laquelle le parfum encapsulé particulaire comprend des capsules ayant une teneur en eau inférieure à 5 %, ayant une enveloppe d'aminoplaste,
un diamètre moyen de particules en masse dans l'intervalle de 25 à 60 μm,
une enveloppe ayant une épaisseur moyenne mesurée dans l'intervalle de 2,5 à 8 μm
un rapport de l'épaisseur d'enveloppe au diamètre moyen de particules dans l'intervalle de 1:3 à 1:20
et une dureté Hysitron, mesurée dans un dispositif de Tribo-indentation Hysitron équipé d'une extrémité Berkovich et programmé pour réaliser une indentation par compression d'un échantillon avec une forme de contact initiale de 75 μN, pendant 10 secondes, suivie par une étape de maintien de position pendant 1 seconde et une étape de décompression pendant 10 secondes, dans l'intervalle de 1,5 MPa à 50 MPa.

2. Composition selon la revendication 1, dans laquelle l'aminoplaste est dérivé de mélamine.

3. Composition selon l'une quelconque des revendications précédentes, dans laquelle l'aminoplaste est dérivé de formaldéhyde.

4. Composition selon l'une quelconque des revendications précédentes, dans laquelle les capsules présentent une épaisseur moyenne d'enveloppe dans l'intervalle de 4 à 8 μm.

5. Composition selon l'une quelconque des revendications précédentes, dans laquelle les capsules présentent un rapport taille moyenne de particules : épaisseur d'enveloppe dans l'intervalle de 5:1 à 10:1.

6. Composition selon la revendication 5, dans laquelle le rapport taille moyenne de particules : épaisseur d'enveloppe se trouve dans l'intervalle de 6,5:1 à 9:1.

7. Composition selon l'une quelconque des revendications précédentes, dans laquelle les capsules présentent un volume moyen de noyau de 35 à 55 % en volume.

8. Composition selon l'une quelconque des revendications précédentes, dans laquelle les capsules présentent une dureté Hysitron dans l'intervalle de 2,5 à 4 MPa.

9. Composition selon l'une quelconque des revendications précédentes qui contient de 0,1 à 4 % en masse des capsules.

10. Composition selon l'une quelconque des revendications précédentes qui contient de plus un parfum non-encapsulé.

11. Composition selon l'une quelconque des revendications précédentes, dans laquelle la combinaison comprend de 30 à 70 % en masse/masse d'huile de silicone volatile et de 20 à 40 % en masse/masse d'huile d'ester et/ou d'huile d'éther.

12. Composition selon la revendication 11, dans laquelle au moins 90 % en masse/masse de l'huile de silicone volatile est du cyclopentadiméthicone et/ou cyclohexadiméthicone.

13. Composition selon la revendication 11, dans laquelle l'huile d'ester est un benzoate d'alkyle et éventuellement une huile de triglycéride d'un acide gras en $C_{18}$ insaturé.

14. Composition selon l'une quelconque des revendications précédentes contenant au moins un gélifiant choisi parmi des cires, des polymères solubles dans l'huile et des gélifiants formant des fibres non-polymères à une concentration

en masse dans la composition choisie dans l'intervalle de 1,5 à 30 % et la composition ayant une dureté de pénétration de 7 à 13 mm.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 2274989 A, Firmenich & Cie
- US 3516941 A
- EP 1797947 A
- US 4278658 A
- EP 6739 A, Unilever NV
- US 3792068 A, Luedders
- WO 9827954 A
- US 6410003 B
- US 7332153 B
- US 6410001 B
- US 6321841 B
- US 6248312 B
- US 5500209 A
- US 6353076 B
- US 6616921 B
- EP 0303461 A
- EP 1289484 A
- US 4232977 A
- US 4605330 A
- WO 09818695 A
- WO 09603899 A
- WO 09405180 A
- WO 09325113 A
- WO 09305678 A
- EP 1040445 A
- US 5997202 A
- US 5897263 A
- US 5496122 A
- US 5275496 A
- US 6598767 B
- US 6299369 B
- WO 200203830 A
- US 4865231 A
- US 5000356 A
- US 6116803 A
- US 5961007 A
- WO 9851185 A
- EP 0312165 A
- WO 0019860 A
- EP 0709041 A
- EP 858271 A
- US 5573341 A
- US 5725133 A
- US 5248213 A
- US 6398439 B
- US 6450716 B